# EUROPEAN PATENT APPLICATION

(11) **EP 4 066 725 A1**
(43) Date of publication of application: **05.10.2022**
(21) Application number: 22163486.8
(22) Date of filing: 22.03.2022
(51) Int. Cl.: A61B 1/018, A61B 17/34, A61B 1/00

(54) **PROTECTION DEVICE FOR AN ENDOSCOPIC APPARATUS**

(30) Priority: 30.03.2021 IT 202100007793
(71) Applicant: Bidoia Medica Sas Di Bidoia Gianfranco, 35010 Vigonza (PD) (IT)
(72) Inventor: BIDOIA, Gianfranco, 35142 PADOVA (IT)
(74) Representative: Modiano, Micaela Nadia

(57) **Abstract**

A protection device (10) for an endoscopic apparatus, which comprises a handpiece (11) from which a self-supporting and rigid cannula (12) protrudes and is joined, inside the handpiece (11), to a part (29) with a through hole and which is at least partially made of elastically extensible plastic material.

## Description

The present invention relates to a protection device for an endoscopic apparatus.

The invention can be applied in the medical sector, in the field of diagnostics and of endoscopic surgery, in particular in gynecology and urology, but it can however also be applied in other branches of medicine, such as for example laparoscopy.

Nowadays diagnostic and surgical techniques are known and increasingly widespread which involve the use of endoscopic apparatuses provided with optical systems and instruments of very small dimensions, even in the order of millimeters.

These apparatuses are used owing to the necessity to carry out tests or operations that are minimally invasive for the patient.

Normally, these apparatuses comprise a probe comprising a shaft containing a bundle of optic fibers and an end optical element, generally a lens, and is adapted to be inserted into the body of the patient.

Generally this probe is connected, at an opposite end from the end with the optical element, to an image display and acquisition system, such as, for example, a video camera.

In some applications, the endoscopic apparatus involves the use of a cannula, for containing the probe, which serves to direct the probe toward the target organ.

In this type of apparatus, the connecting element between the cannula and the image acquisition system is tubular in extension and comprises a plurality of openings that are interfaced: to a lighting system; to one or more systems for administering fluids, such as physiological saline or sterilized water, which are controlled by valves; to one or more entry routes for the insertion of surgical or diagnostic instruments, such as for example a biopsy probe.

Such systems and instruments use the interspace generated between the internal wall of the cannula and the optical endoscopic probe in order to reach the target organ.

In general, for safety, medical devices such as instruments for medical endoscopy, since they can be used in contact with internal parts of the human body, possibly in the presence of bleeding or following perforations or incisions, must be sterile.

Such instruments can be of two types, based on the materials of which they are made:
- completely made of metal, the components of which are reusable, washable and repeatedly sterilizable using known and standardized methods,
- completely made of plastic material or with some components of metal, in any case single-use and already rendered sterile, using known and standardized methods.

For endoscopy instruments made entirely of metal, the end of the cannula is open, in order to allow the insertion into the body of other instruments, called surgical instruments, or the inside of the body to be viewed, up to the target organ, using optical devices like the probe.

For the instruments to be minimally invasive, the diameters of the tubes carrying the optical devices, which must ensure the illumination of the area to be operated on or examined and at the same time provide the image of that area to the monitor, are inherently very small and extend for lengths that can reach 310 mm. These parts are therefore thin and long, and for this reason they are delicate and sensitive to repeated cleanses, washes and sterilization cycles, which is why they are inserted in parts that are more resistant to sterilization, like the cannula. The cannula, of metal or of plastic material, which comes into contact with the patient and with his or her bodily fluids, must be sterilized after every single use.

This sterilization entails a conspicuous expenditure of time that staff have to devote to this operation and which translates to time for which the apparatus is not in use, with consequent increase in the wait times to carry out another examination or operation, and to considerable costs, not just for staff, but also for the substances used to ensure an adequate cleansing. To these we can further add the initial purchase costs of a number of apparatuses such that availability for use is ensured when some of these are being sterilized.

Instruments made at least partially of plastic material are typically single-use, sometimes even single-patient, and these must also be sterile in order to come into contact with parts subjected to surgery or in order to enter existing cavities of the body or parts that are to be subjected to operations, such as biopsies, diathermocoagulations or other operations that can cause bleeding, including when the target organs are hard to reach owing to the difficulty of introducing the instruments into cavities that are problematic to penetrate.

To this end, apparatuses are nowadays often used with a non-single-use part, for example the optical instrumentation, and a single-use part. In apparatuses for endoscopy, these are typically a single-use kits which have an external cannula with a sheath inside it, both of which are made of plastic material. The sheath has a free end which is adapted to be inserted into the patient, where the optical element of the probe reaches. The closing section of the sheath, at the free end, normally has a beaker-shaped optical element, inserted inside or outside the free end of the sheath and heat-sealed or glued to it. The optical probe is inserted into the sheath.

This sheath ensures that the probe never comes into contact with the patient and with his or her bodily fluids. It is possible to simply replace the kit after each use of the apparatus without needing to sterilize the optical element, thus reducing the running costs of the apparatus, the time not in use, and the wear of the optical element.

This conventional technique shows room for improvement, however.

Normally the optical probe comprises an end element which is provided with a lens inclined at a 30° angle, and the sheath is made of non-rigid plastic material and with a certain capacity for stretching, such as for example polyurethane, which can easily break during the introduction and/or rotation of the probe.

Furthermore this sheath is shorter than the probe, so that it stretches in length upon the passage of the probe, in so doing thinning the wall and so determining a better flow of light and of images, but the inclined lens often ends up perforating the sheath.

Furthermore, similar probes can have different lengths, both because each maker can vary the lengths from a few tenths of a millimeter to more than a millimeter, and also because any repairs can entail a reduction in length of as much as 4 mm.

These drawbacks have been overcome in the manner taught in Italian patent document no. 102017000106689 filed on 25 September 2017 by this same applicant, wherein the sheath is made of self-supporting plastic material, thus preventing breakages deriving from the use of a sheath of non-rigid material, but it is still adaptable, allowing a partial elastic lengthening.

But the apparatus still has room for improvement.

In fact, the probes are fragile and delicate and their resistance is tested by repeated cleaning cycles and especially by being handled by operators during introduction into difficult cavities, such as the bladder, cranial, cardiac, vaginal and uterine cavities. Therefore it is not unusual for apparatuses with optical elements to be used to dilate or push aside internal parts of the body by directing the cannula laterally in a 360° loop, shifting organs or tissues, sometimes stenotic, that offer resistance, often non-negligible, to such maneuvers. Bending too far can result in the breakage of the shaft of the probe.

It would appear that this drawback can be overcome with the use of a cannula made of partially elastic metallic material, but distorted images would be obtained on the monitor, at least until the cannula returns to its correct alignment and, with it, also the optical system for transmitting light and images.

However, this solution exhibits a not negligible limitation, which is that stainless steel (and other, similar materials) cannot be lengthened to compensate for the different lengths of the probes.

The aim of the present invention is to provide a protection device for an endoscopic apparatus which is capable of improving the known art in one or more of the above mentioned aspects.

Within this aim, an object of the invention is to provide a protection device that is capable of protecting the shaft of the optical probe from breakage owing to excessive bending and at the same time is capable of compensating for different probe lengths.

Another object of the invention is to provide a protection device that is single-use, by virtue of which a bending of the optical probe that could lead to the loss of the image, is prevented.

Another object of the invention is to provide a protection device by virtue of which lengthy and expensive procedures to clean and sterilize the probe, which moreover could lead to damage to the apparatus, are not necessary.

Another object of the invention is to provide a protection device that does not impede and does not distort the transmission of light and of images.

Another object of the present invention is to overcome the drawbacks of the background art in a manner that is alternative to any existing solutions.

Another object of the invention is to provide a protection device that is highly reliable, easy to implement and of low cost.

This aim and these and other objects which will become better apparent hereinafter are achieved by a protection device for an endoscopic apparatus, characterized in that it comprises a handpiece from which a self-supporting and rigid cannula protrudes, said cannula being coupled, inside said handpiece, to a part with a through hole and which is at least partially made of elastically extensible plastic material.

This aim and these and other objects which will become better apparent hereinafter are also achieved by a protection device for an endoscopic apparatus, characterized in that it comprises a self-supporting and rigid cannula which is coupled to a part with a through hole and which is at least partially made of elastically extensible plastic material, and a tubular covering on the outside of said cannula.

Further characteristics and advantages of the invention will become better apparent from the detailed description that follows of a preferred, but not exclusive, embodiment of the protection device according to the invention, which is illustrated, by way of non-limiting example, in the accompanying drawings wherein:

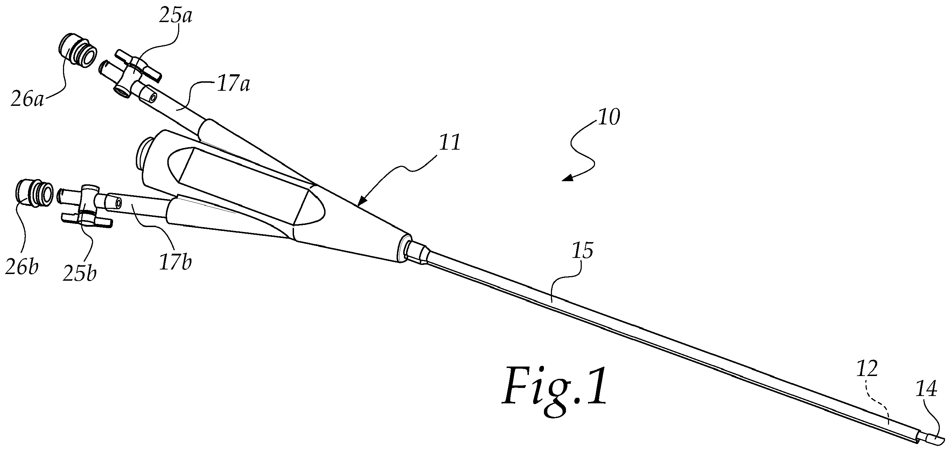

- Figure 1 is a perspective view of a protection device according to the invention;

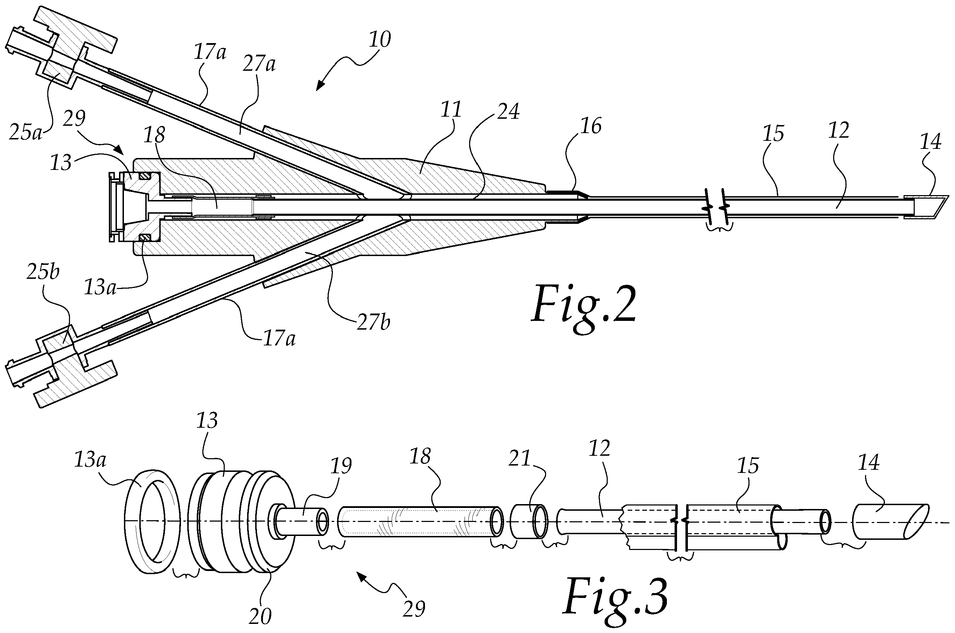

- Figure 2 is a cross-sectional view of the protection device according to the invention;
- Figure 3 is an exploded view of a part of the device according to the invention,
- Figure 4 is a cross-sectional view of a portion of the device according to the invention;
- Figure 5 is a cross-sectional view of another portion of the device according to the invention;
- Figure 6 is a cross-sectional view of the device taken along the line VI-VI indicated in Figure 5.

With reference to the figures, the protection device for an endoscopic apparatus, according to the invention, is generally designated by the reference numeral 10.

The device 10 comprises a handpiece 11 made of plastic material which enables the healthcare worker to maneuver the endoscopic apparatus. The handpiece 11 has an elongated box-like shape and a self-supporting and rigid cannula 12, made of metallic material which is preferably stainless steel, protrudes from it, at one end. The cannula 12 is joined, inside the handpiece 11, to a part 29 with a through hole and which is at least partially made of elastically extensible plastic material. In particular, such part 29 comprises an end element 13 with a through hole and a sleeve 18 for connection between the cannula 12 and the end element 13. The sleeve 18 is made of elastically extensible plastic material, preferably silicone rubber. The end element 13 is inserted into an end section of the handpiece 11 which is conveniently contoured and protrudes partially therefrom.

In another embodiment, not shown, the part 29 can comprise an end portion with a through hole and a tubular portion that extends from the end portion and at which said cannula 12 is coupled; the end portion and the tubular portion are made monolithically of elastically extensible plastic material. Substantially, the end portion and the tubular portion can be identified with the above mentioned "end element 13" and "sleeve 18", but made in a single piece and of a single material, preferably silicone rubber.

By "self-supporting and rigid" is meant that the cannula 12 is capable of supporting itself and is not flexible, but tends instead to maintain its straight shape if subjected to bending forces.

In the handpiece 11, through the end element 13, the probe of a system for acquiring and viewing images, not shown in the figures, can be inserted and also a lighting device, also not shown in the figures. The system for acquiring and viewing images is connected at the end of the handpiece 11, inside which the end element 13 which acts as a connector is arranged, conveniently with a gasket 13a, preferably an O-ring.

At the free end of the cannula 12 there is a beaker-shaped optical end element 14 which closes the cannula 12 at the distal end of the device. The optical end element 14, which is fitted over and glued to the cannula 12, is made of transparent plastic material and has a base 14a with an inclination comprised between 0° and 30° and, preferably, 30° with respect to the plane perpendicular to the plane of the walls.

By the term "free end" is meant the end arranged opposite from the one inside the handpiece 11.

By the term "distal end" is meant the end of the device that is farthest from the operator, therefore opposite to the handpiece 11 and proximate to the optical end element of the probe.

The device 10 also comprises a tubular covering 15 outside the cannula 12, which extends starting from the handpiece 11, where it is fitted with interference over a perforated shank 16 of the handpiece 11 itself, toward the free end of the cannula 12, therefore toward the optical end element 14. The cannula 12, after insertion of the optical probe, is adapted to protrude with respect to the covering 15.

The outer covering 15 is flexible and is made of plastic material, for example crystalline vinyl material.

In the example of the protection device 10 shown, the handpiece 11 comprises two tubular and flexible connecting appendages 17a and 17b, respectively for the insertion of a system for administering fluids and/or for the insertion of a surgical instrument. The two appendages are identical and mirror-symmetrical and in this manner, according to requirements, it is possible to connect the system for dispensing fluids indifferently to one or the other appendage and it is possible to insert the surgical instrument in the opposite appendage. Other details of the appendages 17a, 17b, which are known per se, are provided in the following description.

The sleeve 18 for connection between the cannula 12 and the end element 13 is fitted over a perforated shank 19 of the latter.

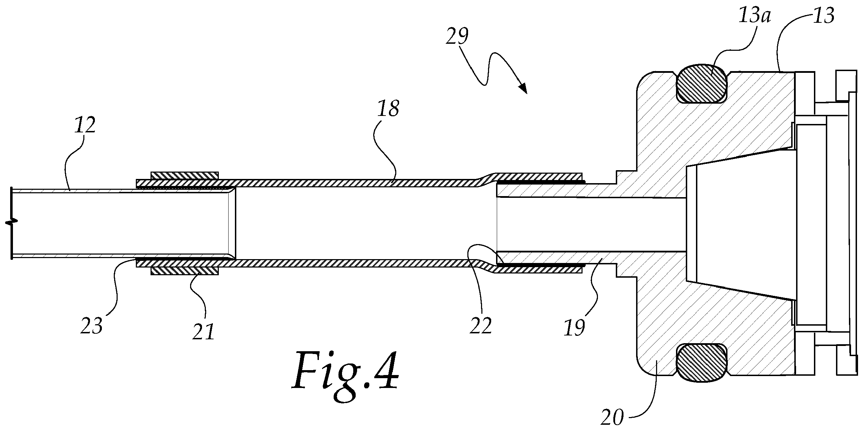

The use of the sleeve 18 is clearly visible in Figure 4.

The end element 13 substantially comprises a perforated flanged portion 20, on one side of which the vision system and the lighting device can be associated, and the perforated shank 19 extends on the side opposite to this portion. A single hole passes through them both in order to permit the passage of the probe.

At least one portion of the shank 19 is inserted in the sleeve 18 with interference at one end, and an end portion of the cannula 12 is inserted in the sleeve, again with interference, at the opposite end.

Still with reference to Figure 4, it can be seen that the device 10 can also comprise a band 21 around the superimposed parts for the interference coupling of the sleeve 18 and of the cannula 12.

This band 21 is made of heat-shrink material, in order to better ensure the adherence of the two underlying elements (which are also glued together, as described below) after its application.

The shank 19 of the end element 13 and the sleeve 18 are coupled by interference and with the interposition of adhesive, defining the layer indicated with 22 and constituted preferably by a cyanoacrylic glue.

At the other end, the sleeve 18 and the cannula 12 are coupled by interference and with the interposition of another adhesive, defining the layer indicated with 23 and constituted preferably by epoxy glue.

The two glues, cyanoacrylic and epoxy, can be mixed together for one or for both of the adhesive bonding seats.

The cannula 12, which, as already said, is made of metallic material, in order to improve the grip of the glue thereupon, has a rough outer surface in the coupling region, preferably knurled, as well as a camber angle at its end, in order to facilitate the introduction of the optical probe.

Instead of adhesives, the couplings between the same parts can be obtained via the use of ultrasound systems.

The sleeve 18 can in fact be coupled to the end element 13 and to the cannula 12 with joining techniques without glues, such as ultrasound, heat-sealing and the like.

The two appendages 17a and 17b merge in a conventional manner into a primary conduit 24 that passes through the handpiece 11 and the covering 15. Each appendage 17a, 17b is provided with a valve, respectively 25a and 25b, for opening and closing the connector. Conveniently, two respective sealing elements 26a and 26b are provided at the free ends of the appendages 17a, 17b. These appendages 17a, 17b extend along convergent coplanar directions, defining two secondary conduits 27a, 27b which converge in a common zone with the primary conduit 24.

The cannula 12 acts as a guide both for the probe and for a surgical instrument, inserted from one of the appendages, toward the target organ.

The secondary conduits 27a and 27b lead inside the handpiece 11, in the primary conduit 24, so as to dispense the fluids used to actuate the apparatus inside the covering 15 and, at the same time, so as to allow the insertion of the surgical instrument.

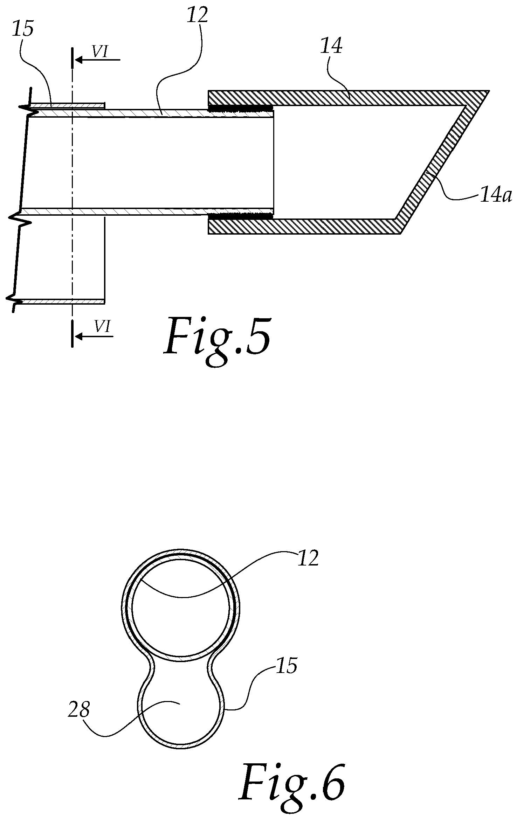

The play deriving from the difference between the outside diameter of the cannula 12 and the inner diameters of the handpiece 11 first and then of the outer covering 15 determines the formation of an interspace 28 that guides the fluids and/or the surgical instruments to the target organ, proximate to the optical element 14. Figure 6 shows a cross-section of the device 10 in which a part of interspace 28 can be seen in the area outside the handpiece 11.

The interspace 28 is closed proximate to the distal end by the presence of the end element 13, with its flanged portion 20 and with the gasket 13a which ensures the seal.

The invention also relates to a protection device for an endoscopic apparatus, which comprises the self-supporting and rigid cannula 12, joined to the part 29 that is provided with a through hole and which is at least partially made of elastically extensible plastic material. The device also comprises the tubular covering 15 outside the cannula 12.

The use of the protection device according to the invention is evident from the foregoing description and explanation and, in particular, it is evident that with the rigidity of the cannula any bending is prevented including the bending of the probe of the image display and acquisition system, therefore keeping light and vision correct and undistorted, and therefore also eliminating almost completely the probability of breakage of the probe during its use. Furthermore, it is also evident that the end element 13, connected even if indirectly to the cannula 12, makes it possible to easily compensate for the different lengths of the various optical probes that can be used (and without interfering with the transmission of light and of images), by virtue of the material of which it is made which renders it elastically extensible.

In practice it has been found that the invention fully achieves the intended aim and objects by providing a single-use protection device that is capable of protecting the shaft of the optical probe from breakage and at the same time is capable of compensating for the different lengths thereof, thus safeguarding the availability and the quality of the images.

The invention thus conceived is susceptible of numerous modifications and variations, all of which are within the scope of the appended claims. Moreover, all the details may be substituted by other, technically equivalent elements.

In practice the materials employed, provided they are compatible with the specific use, and the contingent dimensions and shapes, may be any according to requirements and to the state of the art.

The disclosures in Italian Patent Application No. 102021000007793 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A protection device for an endoscopic apparatus, **characterized in that** it comprises a handpiece (11) from which a self-supporting and rigid cannula (12) protrudes, said cannula (12) being coupled, inside said handpiece (11), to a part (29) with a through hole and which is at least partially made of elastically extensible plastic material.

2. The device according to claim 1, **characterized in that** said part (29) comprises an end element (13) with a through hole and a sleeve (18) for connection between said cannula (12) and said end element (13), said sleeve (18) being made of elastically extensible plastic material.

3. The device according to claim 1, **characterized in that** said part (29) comprises an end portion with a through hole and a tubular portion that extends from said end portion and at which said cannula (12) is coupled, said end portion and said tubular portion being made monolithically of elastically extensible plastic material.

4. The device according to one or more of the preceding claims, **characterized in that** it comprises a tubular covering (15) on the outside of said cannula (12), which extends starting from said handpiece (11) toward the free end of said cannula (12).

5. The device according to one or more of the preceding claims, **characterized in that** said sleeve (18) for connection between said cannula (12) and said end element (13) is fitted over a shank (19) of the latter.

6. The device according to one or more of the preceding claims, **characterized in that** at least one portion of said shank (19) is inserted in said sleeve (18) with interference at one end, and an end portion of said cannula (12) is inserted in said sleeve, again with interference, at the opposite end.

7. The device according to one or more of the preceding claims, **characterized in that** it comprises a band (21) around the superimposed parts for the interference coupling of said sleeve (18) and of said cannula (12).

8. The device according to one or more of the preceding claims, **characterized in that** said band (21) is made of heat-shrink material.

9. The device according to one or more of the preceding claims, **characterized in that** said shank (19) and said sleeve (18) are coupled by interference and with the interposition of adhesive.

10. The device according to one or more of the preceding claims, **characterized in that** said sleeve (18) and said cannula (12) are coupled by interference and with the interposition of adhesive, said cannula (12) being made of metallic material and having a rough external surface, at least in the coupling region.

11. The device according to one or more of the preceding claims, **characterized in that** said adhesive between said shank (19) and said sleeve (18) is constituted by a cyanoacrylic glue.

12. The device according to one or more of the preceding claims, **characterized in that** said adhesive between said sleeve (18) and said cannula (12) is constituted by an epoxy glue.

13. The device according to one or more of the preceding claims, **characterized in that** said sleeve (18) is coupled to said end element (13) and to said cannula (12) with at least one joining method chosen between ultrasound and heat-sealing.

14. A protection device for an endoscopic apparatus, **characterized in that** it comprises a self-supporting and rigid cannula (12) which is coupled to a part (29) with a through hole and which is at least partially made of elastically extensible plastic material, and a tubular covering (15) on the outside of said cannula (12).
